# EUROPEAN PATENT APPLICATION

(11) **EP 1 618 891 A1**
(43) Date of publication of application: **25.01.2006**
(21) Application number: 04724749.9
(22) Date of filing: 31.03.2004
(51) Int. Cl.: A61K 45/00, A61K 39/395, A61P 37/06, C07K 16/18, C07K 16/46, C12P 21/08, C12Q 1/02

(54) **METHOD OF INDUCING DIFFERENTIATION AND PROLIFERATING REGULATORY T CELL BY ANTI-CD52 ANTIBODY AND MEDICINAL COMPOSITION THEREFOR**

(30) Priority: 31.03.2003 JP 2003095765; 11.12.2003 JP 2003413786
(71) Applicant: KIRIN BEER KABUSHIKI KAISHA, Tokyo 104-8288 (JP); Masuyama, Junichi, Oyama-shi, Tochigi 323-0829 (JP)
(72) Inventor: MASUYAMA, J., Oyama-shi, Tochigi 323-0829 (JP); WATANABE, T., Pharmaceutical Research Laboratories, Takasaki-shi, Gunma 370-1295 (JP); SOMA, Y., Pharmaceutical Research Laboratories, Takasaki-shi, Gunma 370-1295 (JP); YAMAGUCHI, Y., Pharmaceutical Division, Shibuya-ku, Tokyo 150-8011 (JP)
(74) Representative: HOFFMANN EITLE
(86) International application number: PCT/JP2004/004698
(87) International publication number: WO 2004/087210

(57) **Abstract**

An object of the present invention is to provide a method for inducing differentiation and/or promoting proliferation of regulatory T cells, a method for suppressing the transendothelial cell migration of immunocytes, a method for suppressing immunity using these methods, and a pharmaceutical composition to be used for these methods. Provided are a method for inducing differentiation and/or promoting proliferation of regulatory T cells by causing CD52 agonists, including anti-CD52 antibodies, to act on CD52-expressing T cells, a method for suppressing the transendothelial cell migration of immunocytes, and a method for suppressing immunity using these methods.

## Description

### TECHNICAL FIELD

The present invention relates to a novel method for suppressing immunity, particularly a method for suppressing immunity by inducing differentiation and/or promoting proliferation of regulatory T cells (which may also be referred to as suppressive T cells), and a pharmaceutical composition used for such method.

### BACKGROUND ART

We have discovered that a 4C8 monoclonal antibody (4C8 mAb) that inhibits the transendothelial migration of human T cells after such cells adhere to vascular endothelial cells (Masuyama, J. et al., (1999) J. Exp. Med., 189, 979). For T cells to travel to peripheral tissues such as that containing inflammation, a process is necessary whereby T cells firmly adhere to vascular endothelial cells via integrin molecules, morphologically change to become appropriate for cell movement, and then migrate out through spaces between vascular endothelial cells. It is known that not all the T cells that have adhered to vascular endothelial cells migrate subendothelially, but CD4-positive CD45RO-positive CD26-strongly-positive activated memory T cells selectively migrate. Specifically, 4C8 mAb is a monoclonal antibody that does not suppress T cells from adhering to vascular endothelial cells and specifically suppresses T-cell-subset-selective transendothelial migration.

Furthermore, ability to induce regulatory T cells has been reported as a new function of 4C8 mAb (Masuyama, J. et al., (2002) J. Immunol., 169, 3710). When T cells are stimulated with a CD3 agonist having a low concentration such that the CD3 agonist alone is incapable of activating T cells, T cells can be activated by the simultaneous application of appropriate costimulation. Such costimulation not only enhances the activation ability of the CD3 agonist, but also has a significant effect on the functions of T cells after activation. When OKT3, which is an anti-CD3 antibody, was used as a CD3 agonist, stimulation with 4C8 mAb functioned as costimulation, thereby activating CD4-positive T cells and causing the T cells to proliferate. Furthermore, cells activated by simultaneous stimulation with CD3 and 4C8 mAb showed *in vitro* suppressive activity upon the proliferation of CD4-positive T cells due to polyclonal stimulation, revealing that regulatory T cells had been induced.

### 1. Regulatory T cell

T cells form one of the cell groups that play a central role in the immune system as a host defense system against various pathogens. T cells are largely classified into CD4-positive helper T cells and CD8-positive cytotoxic T cells. The former T cells can be mainly classified into Th1 cells that produce IFN-γ, Th2 cells that produce IL-4, and the like based particularly on cytokine production patterns at specific differentiation and maturation stages after stimulation with antigens. Generally, the former T cells are deeply involved in host defense as cells for cell-mediated immunity and the latter T cells are deeply involved in the same as cells for humoral immunity. Immune responses are deeply involved in the elimination of pathogens and the acquisition of resistance to infection through the functions of such T cells having different properties under a delicate balance. In general, it is known that in healthy immune responses, mechanisms work to eliminate foreign non-autoantigens, but elimination mechanisms do not work against autoantigens forming a living body because immunological tolerance has been established. However, excessive immune responses to autoantigens result in the development of autoimmune disease. Hence, immunological tolerance for autoantigens is not absolute. However, at the T cell level, mechanisms by which various types of immunological tolerance are induced are known. One type of immunological tolerance is a mechanism for eliminating self-reactive T cell clones in the thymus, which is referred to as central tolerance (Kisielow, P. et al., 1988, Nature 333: 742-746.). The other type is a mechanism referred to as peripheral tolerance by which self-reactive T cells outside the thymus are controlled. As the latter mechanism, mechanisms for cell death induction or induction of anergy to autoantigens (Rocha, B., and H. von Boehmer, 1991, Science 251: 1225-1228; Jenkins, M. K. and R. H. Schwartz, 1987. J. Exp. Med. 165: 302-319) and mechanisms for active suppression (Shevach, E.M., 2000, Annu. Rev. Immunol. 18: 423-449) by regulatory T cells are known. The "regulatory T cell" is a new concept that has been recently proposed, and is used to define a T cell that has suppressive action against other T cells. Immune responses are established under a delicate balance. For example, it becomes known that the above Th1 cells and Th2 cells function antagonistically upon the immune responses of each other; that is, one acts as a regulatory T cell for the other. In the meantime, verification of the presence of a cell population made up of regulatory T cells and property analysis thereof are very controversial in the recent history of immunology. Such regulatory T cells have been studied as cells having functions to suppress or regulate specific immune responses *in vitro* or *in vivo.* As a result, regulatory T cells have been reported as members of various cell populations based on cell surface markers, types of produced cytokines, suppressive and/or regulatory mechanisms, and the like (Roncarolo, M. G. and M. K. Levings, 2000, Curr. Opinion. Immunol. 12: 676-683).

Among these regulatory T cells, recently, the most studied cell population is a T cell population for which a marker is being CD4-positive and CD25-positive as described below. Such T cell population has been mainly studied in species other than humans, such as mice and rats. Specifically, it has been revealed that organ-specific autoimmune disease is induced by removing CD25-positive cells, RT6.1-positive cells (as expressed in the case of most rat mature T cells), CD5 strongly-positive cells, or CD45RB weakly-positive (mouse) or CD45RC weakly-positive (rat) cells from normal mouse or rat CD4-positive spleen cells, and then by transferring the remaining T cells into immunodeficient animals. No such regulatory T cell-specific markers have yet been discovered. The above markers cannot be directly associated with the functions of regulatory T cells and are merely markers indicating the state of activated cells, the state of cells stimulated with antigens, or the memory state. However, by using as an index the possession of not only a function for inducing organ-specific autoimmune disease in immunodeficient animals, but also the possession of a function for suppressing autoimmune disease and autoimmune inflammation by transfer of a specific cell population, the analysis of regulatory T cell populations has been advanced. Thus, it has become known that a T cell population positive to both CD4 and CD25 can be a marker of regulatory T cells (Sakaguchi, S., et al., 1985, J. Exp. Med. 161: 72; Itoh, M., et al., 1999, J. Immunol. 162: 5317-5326; Sakaguchi. S. et al., 1995, J. Immunol. 155: 1151-1164; Asano, M. et al., 1996, J. Exp. Med. 184: 387-396; Read, S. et al., 2000, J. Exp. Med. 192: 295-302; Salomon, B. et al., 2000, Immunity 12: 431-440; Stephens, L. A., and D. Mason, 2000, J. Immunol. 165: 3105-3110).

As described above, CD4-positive CD25-positive regulatory T cells have been identified in mice and rats. However, the presence of similar cells in humans was reported at last in 2001 by several groups (Jonuleit, H. et al., 2001, J. Exp. Med. 193: 1285-1294; Levings, M. K. et al., 2001, J. Exp. Med. 193: 1295-1301; Dieckmann, D. et al., 2001, J. Exp. Med. 193: 1303-1310; Taama, L. S. et al., 2001, Eur. J. Immunol. 31: 1122-1131; Stephens, L. A. et al., 2001, Eur. J. Immunol. 31: 1247-1245; Baecher-Allan, C. et al., 2001, J. Immunol. 167: 1245-1253). These reports are based on the fact that the results obtained using expression of CD4 and CD25 (known in mice) as an index and using cell populations separated from human peripheral blood are equivalent to results that have been reported concerning mice, in terms of various cell surface markers, anergy to stimulation for cell activation, types of produced cytokines, an *in vitro* function to suppress normal T cell proliferation and the mechanism thereof, and the like. Specifically, the CD4-positive CD25-positive T cells separated from human peripheral blood express CD45RO-positive memory T cell markers and express at high levels activation markers such as HLA-DR compared with CD4-positive CD25-negative T cells. In addition, the CD4-positive CD25-positive T cells constantly express CTLA-4 within themselves, and the expression of CTLA-4 increases by stimulation. Furthermore, DNA synthesis of and cytokine production by CD4-positive CD25-positive regulatory T cells are not observed with stimulation with anti-CD3 antibodies, stimulation with anti-CD3 antibodies and anti-CD28 antibodies, stimulation with allogeneic mature dendritic cells (allogeneic mature DCs), or the like. The T cells are in a state of being anergic to stimulation with antigens. Stimulation with cytokines such as IL-2, IL-4, IL-15, or the like in addition to simulation with anti-CD3 antibodies and anti-CD28 antibodies enhances the DNA synthesis ability of the CD4-positive CD25-positive regulatory T cells. But the enhanced DNA synthesis ability is not equivalent to that of CD4-positive CD25-negative T cells. When CD4-positive CD25-negative T cells are stimulated with the anti-CD3 antibodies or the allogeneic mature dendritic cells (allogeneic mature DCs) in the presence of CD4-positive CD25-positive regulatory T cells, CD4-positive CD25-positive regulatory T cell number-dependent proliferation-suppressing action is observed, compared with stimulation in the absence of the CD4-positive CD25-positive regulatory T cells. CD4-positive CD25-positive regulatory T cells have ability, which is lower than that of such cells of mice, to produce suppressive cytokines such as IL-10 and TGFβ1. It has been reported that proliferation-suppressing action on CD4-positive CD25-negative T cells is not abrogated by the action of neutralization antibodies against these cytokines. It has also been reported that such suppressive action requires direct cell-to-cell contact between CD4-positive CD25-negative T cells and CD4-positive CD25-positive regulatory T cells. In mice, rats, and humans, the presence of CD4-positive CD25-positive regulatory T cells has been reported and property analysis therefor has been advanced. However, detailed differentiation mechanisms and mechanisms for suppressive action of these cells are to be elucidated. No specific markers therefor have yet been discovered.

Moreover, it has also been reported that in mice and humans, regulatory T cells are induced by stimulation with allogeneic antigens in the presence of IL-10 or repeated stimulation with allogeneic immature dendritic cells (allogeneic immature DCs) (Groux, H. et al., 1997, Nature 389: 737-742; Jonuliet, H. et al., 2000, J. Exp. Med. 192: 1213-1222). Unlike Th1 cells and Th2 cells, these cells are characterized by producing large quantities of IL-10, modest level of TGF-beta 1, IFN-gamma, and IL-5, a low level of IL-2, and no IL-4. These cells are referred to as Tr1 cells. Similar to CD4-positive CD25-positive regulatory T cells, Tr1 cells are also anergic. However, the mechanism of suppressing T cells can be partially explained based on IL-10 or TGF-β1 produced by Tr1 cells. Nevertheless, it remains almost unknown about if Tr1 cells are T cell subsets that are completely different from CD4-positive CD25-positive regulatory T cells or if the two types are the same and differ in terms of differentiation and activation stages.

By the use of the expression of CD4 and CD25, which are regulatory T cell markers known in mice and rats, as an index, CD4-positive CD25-positive T cells have been separated from human peripheral blood and the like. As a result, it was confirmed that human CD4-positive CD25-positive T cells are similar to those of mice and rats by comparing other cell surface markers and functions known in T cells of mice and rats with those of the human T cells. The results suggested the presence of CD4-positive CD25-positive regulatory T cells in humans.

These cells form a rare cell population that accounts for only 5% to 10% of peripheral blood CD4-positive T cells and is anergic to activation and/or proliferation stimulation. In this case, cell proliferation can be promoted by adding stimuli with cytokines such as IL-2, IL-4, or IL-15 to stimulation with anti-CD3 antibodies and anti-CD28 antibodies. However, such amplification is not sufficient level for clinical applications, such as amplifying the cell count and transferring such cells into humans.

In animal experiments, regulatory T cells function to suppress autoimmune disease through transfer of these cells into animals, and they function to suppress transplantation rejection and graft versus host disease (GvHD) (Hara, M. et al., 2001, J. Immunol. 166: 3789-3796; Taylor, P. A. et al., 2001, J. Exp. Med. 193: 1311-1317). Accordingly, it is possible to apply regulatory T cells for treatment for autoimmune disease, organ transplantation, and the like with cell medicine utilizing the immunosuppressive action of regulatory T cells. The development of a therapy comprising proliferating regulatory T cells using a pharmaceutical composition for promoting regulatory T cell proliferation or by treating *ex vivo* peripheral blood or marrow cells collected from a patient or another human and then returning the cells into the body of a patient has been expected.

### 2. 4C8 antigen

The 4C8 antigen is expressed on some of immune system cells. Such antigen is originally discovered during identification of molecules involved in transendothelial migration of human T cells after their adhesion to vascular endothelial cells. Through screening of monoclonal antibodies obtained by immunization of mice with human T cells using suppression of *in vitro* extravasation of T cells as an index, monoclonal antibodies that recognize 4C8 antigen has been obtained (Masuyama, J. et al., 1999, J. Exp. Med. 189: 979-989; International Patent Publication WO99/12972). For T cells to travel to peripheral tissues such as that containing inflammation, a process is necessary whereby T cells firmly adhere to vascular endothelial cells via integrin molecules, morphologically change so as to become appropriate for cell movement, and then migrate out through the spaces between vascular endothelial cells. It is known that not all T cells that have adhered to vascular endothelial cells migrate subendothelially, but CD4-positive CD45RO-positive CD26-strongly-positive activated memory T cells selectively migrate. Specifically, anti-4C8 monoclonal antibodies (mAb) do not suppress adhesion of T cells to vascular endothelial cells, but are mAbs that specifically suppress T-cell-subset-selective transendothelial migration. It is thought that the 4C8 antigen is a functional molecule essential for such migration. According to verification of the expression of the 4C8 antigen using the 4C8 mAb, the 4C8 antigens are strongly expressed in CD3-positive T cells and are also expressed in B cells, NK cells, monocytes, and eosinophils. However, they are not expressed in neutrophils and endothelial cells. Crosslinking with 4C8 mAb not only promotes actin polymerization of T cells so as to provide cell morphology with polarity, but also stimulates cell movement.

### 3. CD52 (campath-1 antigen)

Campath-1 was discovered by Waldmann et al., as a rat antibody capable of removing lymphocytes from human marrow grafts (Hale, G. et al. (1983) Blood, 62, 873). An antigen (CD52) for Campath-1 had remained unknown for a long time and was then identified in 1991 by Xia et al., as a novel molecule (Xia, M. Q. et al., (1991) European J. Immunol., 21, 1677). Furthermore, it was reported in 1993 that HE5, which had been identified by Kirchhoff et al., as an epithelial specific molecule of the male genital duct, is the same molecule as CD52 (Kirchhoff, C. et al. (1993) Molecular Reproduction and Development, 34, 8).

Campath-1 has action to remove lymphocytes by binding to CD52, which is expressed at high levels on lymphocytes, so as to induce cell death by complements or antibody-dependent cellular cytotoxicity (ADCC). As a very efficient lymphocyte-removing antibody, the application of the antibody in the removal of lymphocytes from marrow grafts for the purpose of alleviating GvHD and application of the same in lymphoma treatments have been actively studied. Campath (trademark) (Alemtuzumab), which is Campath-1H prepared by humanization of a rat antibody Campath-1G, has been marketed as a therapeutic drug for B cell lymphoma by Millennium Pharmaceuticals, Inc. (Pangolis GA et al., (2001) Medical Oncology, 18, 99).

However, the functions of CD52, the antigen of Campath-1, have not been revealed to a significant extent. It has been reported that when CD52 is crosslinked with antibodies on T cells, protein phosphorylation analogous to that in a case where an antigen receptor is stimulated is observed (Hederere RA et al., (2000) International Immunology, 12, 505). Furthermore, it has been reported that crosslinking of CD52 with antibodies provides costimulation (in addition to stimulation with an anti-CD3 antibody or an anti-CD2 antibody), thereby enhancing T cell proliferation (Valentin H et al., (1992) Transplantation, 54, 97; Rowan WC et al., (1994) International Immunology, 7, 69). However, what signals are mediated by CD52 and what effects are provided on cell functions by such costimulation with CD52 are unknown.

CD52 is also expressed in the epithelium of the male genital duct as described above, in addition to lymphocytes, monocytes, macrophages, and the like. CD52 expressed in the male genital duct epithelial is released from epithelium cells and then incorporated in the sugar coating on sperm surfaces in the process of sperm maturation. Negative charge of sialic acids of sugar chains added by CD52 is thought to play a role in preventing aggregation of sperm. Furthermore, an example has also been reported that an antibody against CD52 causes infertility. However, the role of CD52 in sperm functions and the like remains almost entirely unknown (Kirchhoff C et al., (2001) Cells Tissues Organs, 168, 93).

CD52 is a peptide consisting of 12 amino acids and binds to a cell membrane via a GPI anchor (Xia, M. Q. et al., (1991) European J. Immunol., 21, 1677). The molecule itself is very small and CD52 has no intracellular domains. Thus, it is difficult to predict the functions of CD52 from its structure. This may be one of causes of the lack of advances in research concerning the functions of CD52.
Patent document 1 International Patent Publication No. WO99/12972
Patent document 2 JP Patent Publication (Kohyo) 2-503514 A (1990)
Non-patent document 1 Masuyama, J. et al., (2002) J. Immunol., 169, 3710
Non-patent document 2 Hale, G. et al., (1983) Blood, 62, 873
Non-patent document 3 Xia, M. Q. et al., (1991) European J. Immunol., 21, 1677
Non-patent document 4 Kirchhoff, C et al., (1993) Molecular Reproduction and Development, 34, 8
Non-patent document 5 Pangolis GA et al., (2001) Medical Oncology, 18, 99
Non-patent document 6 Hederere RA et al., (2000) International Immunology, 12, 505
Non-patent document 7 Valentin H et al., (1992) Transplantation, 54, 97
Non-patent document 8 Rowan WC et al., (1994) International Immunology, 7, 69
Non-patent document 9 Kirchhoff C et al., (2001) Cells Tissues Organs, 168, 93

### SUMMARY OF THE INVENTION

An object of the present invention is to provide a method for inducing differentiation and/or promoting proliferation of regulatory T cells through the use of CD52, a method for suppressing transendothelial cell migration of immunocytes through the use of CD52, and a method for suppressing immunity using these methods and a pharmaceutical composition to be used for these methods, particularly a pharmaceutical composition containing an anti-CD52 antibody as an active ingredient.

We have intensively studied a 4C8 antigen that is recognized by a 4C8 antibody, thereby discovering that the antigen is a CD52 molecule. Based on conventional findings concerning CD52, it has been impossible to predict the involvement of CD52 in the suppression of transendothelial cell migration of activated T cells or induction of regulatory T cells. We have further intensively studied an anti-CD52 antibody. Thus, we have newly discovered that the anti-CD52 antibody, for which only antibody activity of removing lymphocytes from B-cell lymphoma patients and the like has been known, surprisingly has activity of suppressing transendothelial cell migration and activity of inducing regulatory T cells. Furthermore, we have discovered that the anti-CD52 antibody's activity of suppressing the transendothelial cell migration of activated T cells and activity of inducing regulatory T cells can be broadly utilized for immunotherapy. Thus, we have completed the present invention.

The present invention is as follows:
1. A pharmaceutical composition for immunosuppression, which contains as an active ingredient a CD52 agonist other than a 4C8 antibody;
2. A pharmaceutical composition for inducing differentiation and/or promoting proliferation of a regulatory T cell, which contains as an active ingredient a CD52 agonist other than a 4C8 antibody;
3. The pharmaceutical composition according to 2 above, wherein the regulatory T cell has antigen-selective suppressive activity;
4. The pharmaceutical composition according to any one of 1 to 3 above, which further contains a CD3 agonist;
5. The pharmaceutical composition according to 4 above, wherein the CD3 agonist is an anti-CD3 antibody or a fragment thereof;
6. The pharmaceutical composition according to 5 above, wherein the anti-CD3 antibody is a humanized antibody or a human antibody;
7. A pharmaceutical composition for suppressing transendothelial cell migration of an immunocyte, which contains as an active ingredient a CD52 agonist other than a 4C8 antibody;
8. The pharmaceutical composition according to any one of 1 to 7 above, wherein the CD52 agonist is an anti-CD52 antibody or a fragment thereof;
9. The pharmaceutical composition according to 8 above, wherein the anti-CD52 antibody is a humanized antibody or a human antibody;
10. The pharmaceutical composition according to 9 above, wherein the above humanized antibody is a rat humanized antibody Campath-1H;
11. The pharmaceutical composition according to any one of 1 to 10 above, which is used for preventing or treating autoimmune disease, allergic disease, or transplantation immune response;
12. A method for inducing differentiation and/or promoting proliferation of a regulatory T cell, which comprises causing a CD52 agonist other than a 4C8 antibody to act on CD52 that is expressed on the surface of an immunocyte;
13. The method for inducing differentiation and/or promoting proliferation of a regulatory T cell according to 12 above, wherein the regulatory T cell has antigen-selective suppressive activity;
14. The method according to 12 above, wherein the CD52 agonist is an anti-CD52 antibody or a fragment thereof;
15. The method according to 14 above, wherein the anti-CD52 antibody is a humanized antibody or a human antibody;
16. The method according to 15 above, wherein the above humanized antibody is the rat humanized antibody Campath-1H;
17. The method according to 12 above, which further comprises causing a CD3 agonist to act on CD3 that is expressed on the surface of the above immunocyte;
18. The method according to 17 above, wherein the CD3 agonist is an anti-CD3 antibody or a fragment thereof;
19. The method according to 18 above, wherein the anti-CD3 antibody is a humanized antibody or a human antibody;
20. The method according to any one of 12 to 19 above, wherein the above immunocyte is contained in peripheral blood, lymph node, or thymus;
21. The method according to 20 above, wherein the above immunocyte is a T cell;
22. The method according to 21 above, wherein the above immunocyte is a peripheral blood mononuclear cell;
23. The method according to any one of 12 to 22 above, wherein stimulation of an immunocyte with the CD3 agonist and the stimulation of an immunocyte with the CD52 agonist are carried out *ex vivo;*
24. The method according to any one of 12 to 22 above, wherein stimulation of an immunocyte with the CD3 agonist and stimulation of an immunocyte with the CD52 agonist are carried out *in vivo;*
25. A method for producing an anti-CD52 humanized antibody or human antibody to be used for a drug having an immunosuppressive effect, an effect of inducing differentiation and/or promoting proliferation of a regulatory T cell, and/or an effect of suppressing transendothelial cell migration of an immunocyte;
26. A method for screening using interaction with CD52 as an index for a drug having an immunosuppressive effect, an effect of inducing differentiation and/or promoting proliferation of a regulatory T cell, and/or an effect of suppressing transendothelial cell migration of an immunocyte.

This specification includes part or all of the contents as disclosed in the specifications and/or drawings of Japanese Patent Application No. 2003-95765 and Japanese Patent Application No. 2003-413786, which are priority documents of the present application.

### BRIEF DESCRIPTION OF DRAWINGS

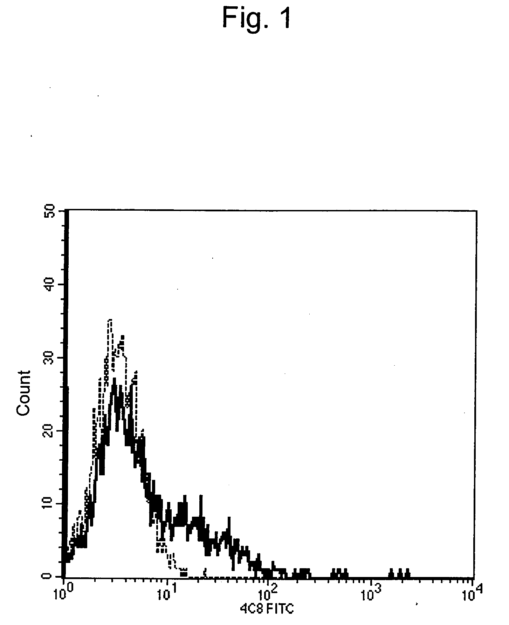

Fig. 1 shows the results of FACS analysis on COS-1 cells having one 4C8-positive clone introduced therein. Bold continuous lines indicate the results of staining with 4C8 mAb and dashed lines indicate the results of staining with an isotype control. Similar results were obtained from other positive clones.

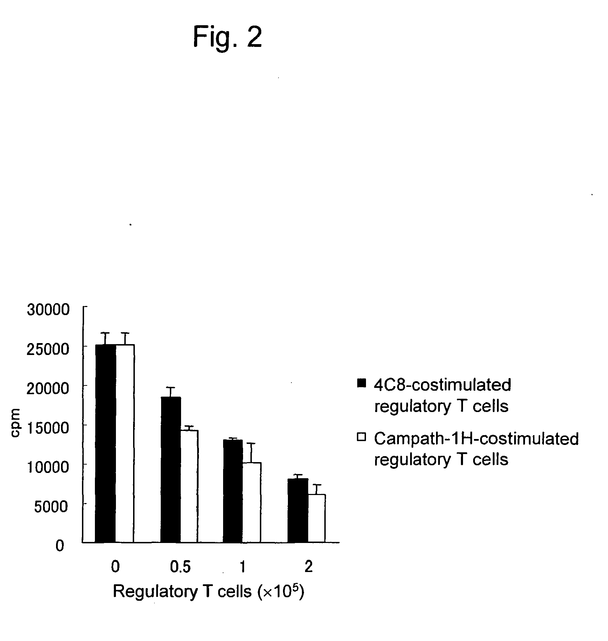

Fig. 2 shows that when cells costimulated with 4C8 mAb or Campath-1H and CD3 were added and then the proliferation of CD4-positive T cells due to stimulation with anti-CD3 antibodies was measured based on incorporation of thymidine, both 4C8 mAb-costimulated cells and Campath-1H-costimulated cells suppressed the proliferation of the CD4-positive T cells due to stimulation with the anti-CD3 antibodies in a cell number dependent manner.

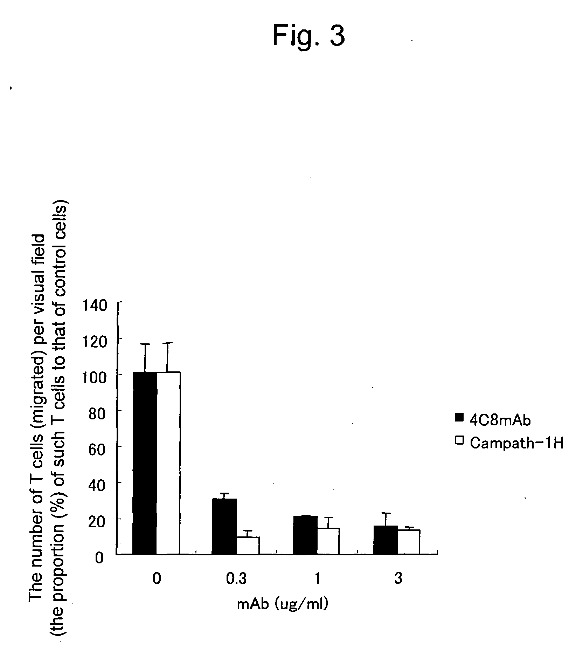

Fig. 3 shows the results of examining the suppression of the migration of CD3-positive T cells into collagen gel under human umbilical vein endothelial cell monolayers by the addition of 4C8 mAb or Campath-1H. Both 4C8 mAb and Campath-1H suppressed the transendothelial cell migration properties of the CD3-positive T cells.

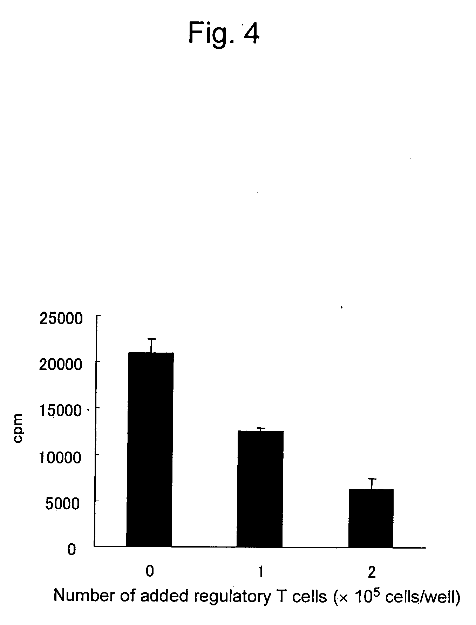

Fig. 4 shows the results of examining the suppression of mixed lymphocyte culture reaction by the 4C8 mAb-costimulated cells. The 4C8 mAb-costimulated cells suppressed the CD4-positive-T-cell-mixed lymphocyte culture reaction in a cell number dependent manner.

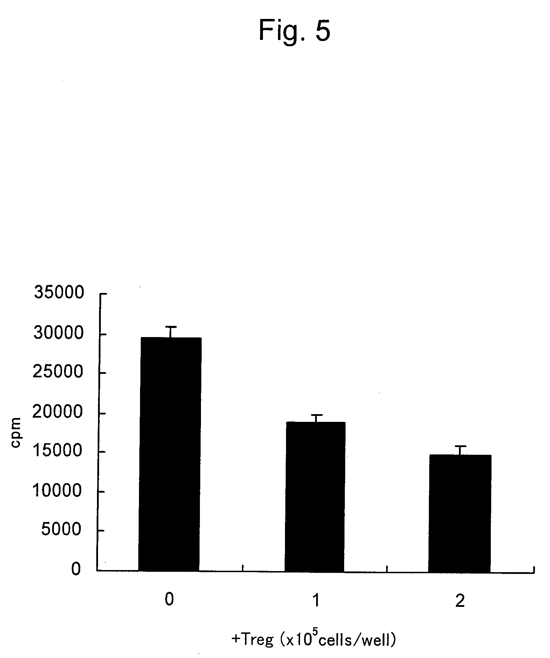

Fig. 5 shows the results of examining the suppression of alloantigen-stimulated CD4-positive T cell reaction by regulatory T cells induced using Campath-1H. The Campath-1H-costimulated cells suppressed the CD4-positive-T-cell-mixed lymphocyte culture reaction in a cell number dependent manner.

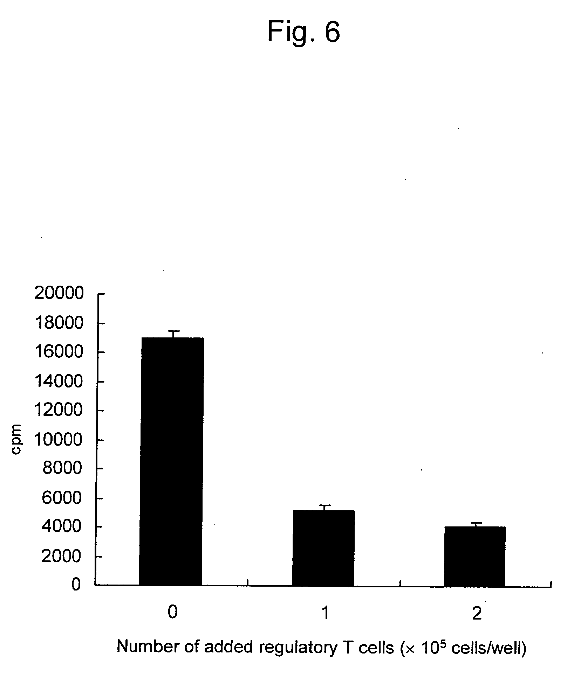

Fig. 6 shows the results of examining the suppression of CD8-positive-T-cell-mixed lymphocyte culture reaction by regulatory T cells induced by costimulation with anti-CD52 antibodies. The 4C8 mAb-costimulated cells suppressed the CD8-positive-T-cell-mixed lymphocyte culture reaction in a cell number dependent manner.

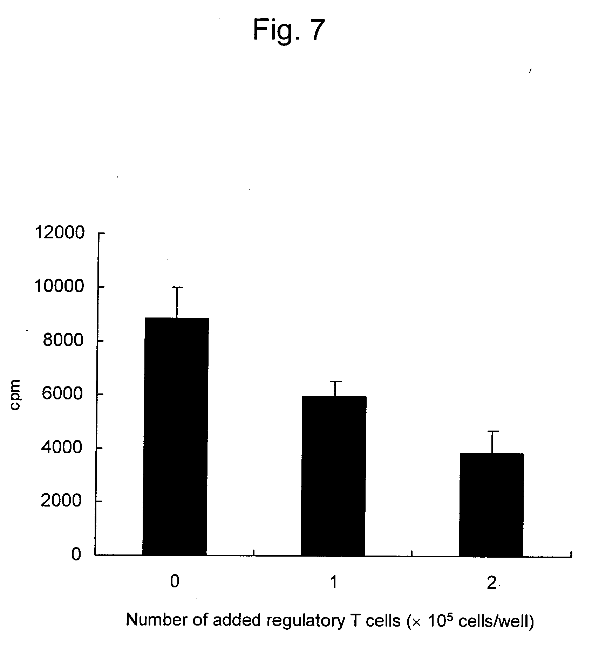

Fig. 7 shows the results of examining the suppression of CD8-positive-T-cell-mixed lymphocyte culture reaction by regulatory T cells induced by costimulation with Campath-1H. Regulatory T cells induced by costimulation with Campath-1H suppressed the CD8-positive-T-cell-mixed lymphocyte culture reaction in a cell number dependent manner.

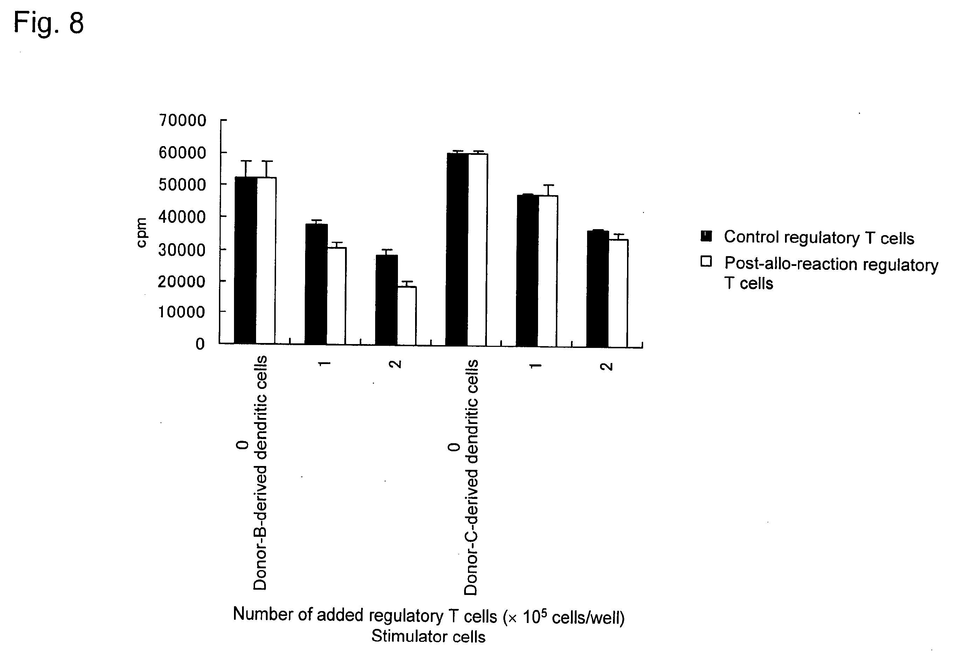

Fig. 8 shows the results of examining the induction of regulatory T cells from CD4-positive T cells after alloantigen reaction. In the reaction to the cells derived from donor B, which is a donor for monocyte-derived mature dendritic cells used in the alloantigen reaction, post-alloantigen-reaction regulatory T cells showed suppressive activity that was stronger than that of control regulatory T cells. However, in the reaction to the cells derived from a third subject, donor C, both suppressive activities were equivalent to each other.

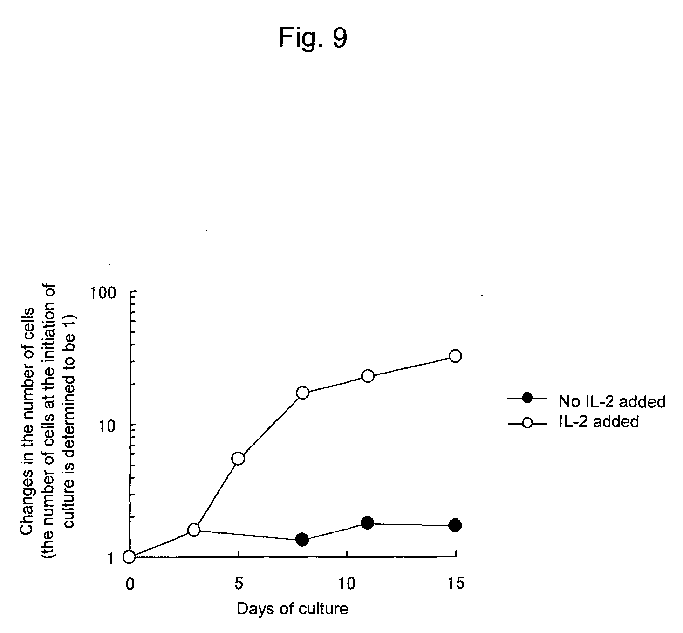

Fig. 9 shows changes in the number of cells at the time of *in vitro* amplification of regulatory T cells induced by costimulation with anti-CD52 antibodies. Due to culture in the presence of IL-2 during days 3 to 8 of culture, amplification of the regulatory T cells was observed.

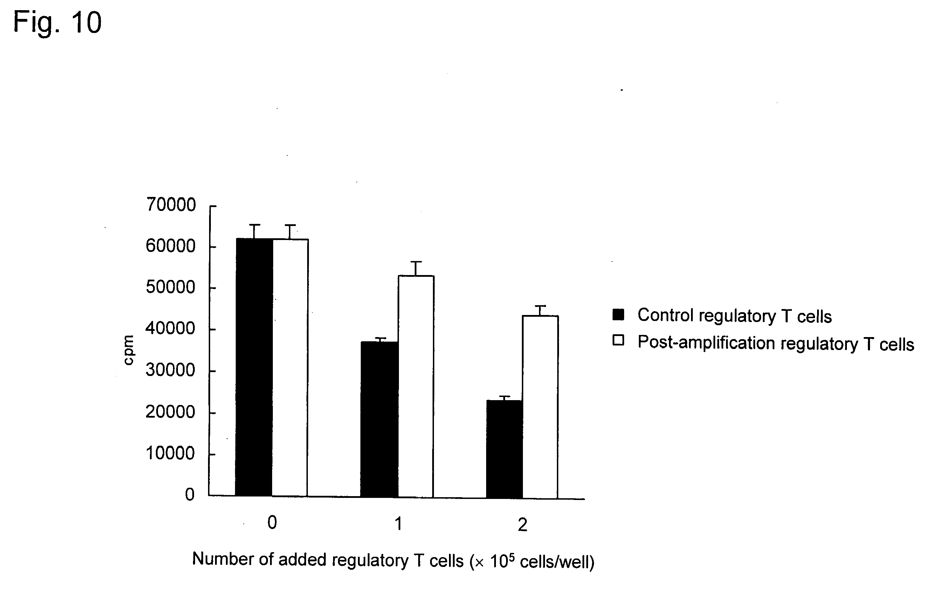

Fig. 10 shows the results of the suppression assay for regulatory T cells on day 8 of culture. Decreased suppressive ability was observed for the thus amplified regulatory T cells.

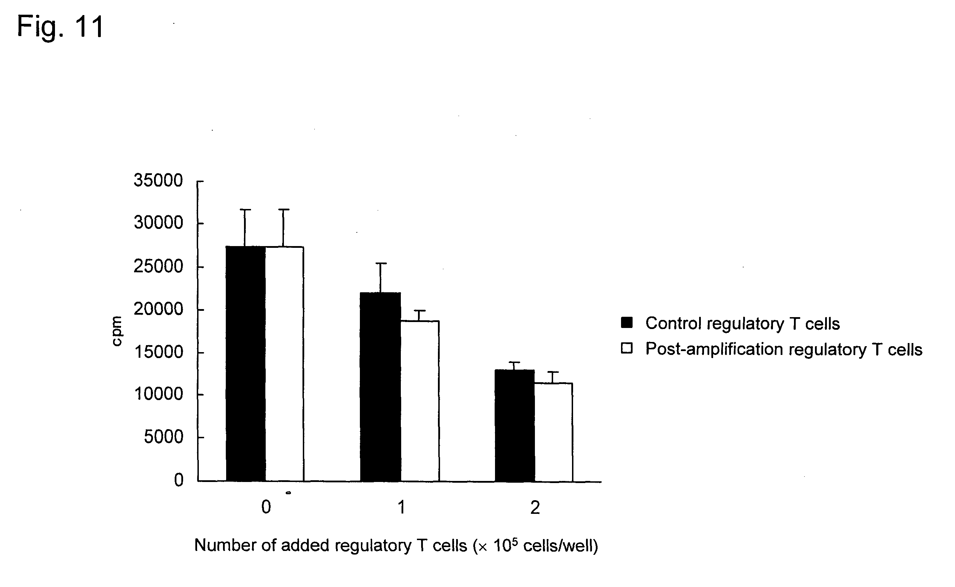

Fig. 11 shows the results of suppression assay for regulatory T cells that had been subjected again to costimulation with anti-CD52 antibodies (day 15). The suppressive ability of the thus amplified regulatory T cells was equivalent to that of control cells.

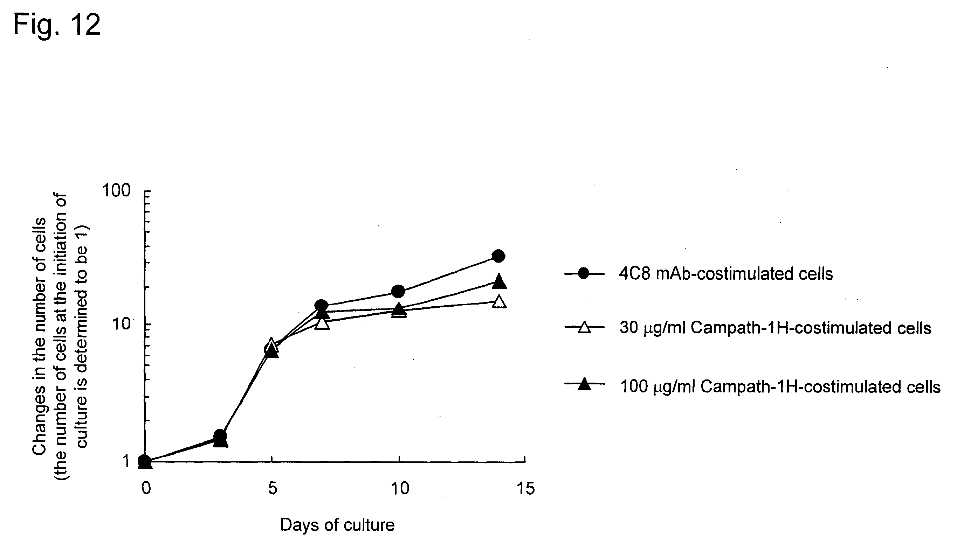

Fig. 12 shows the results of examining *in vitro* amplification of regulatory T cells induced by costimulation with Campath-1H. Due to culture in the presence of IL-2, the Campath-1H-costimulated cells were amplified 10-fold or more in a manner similar to the case of the 4C8 mAb-costimulated cells.

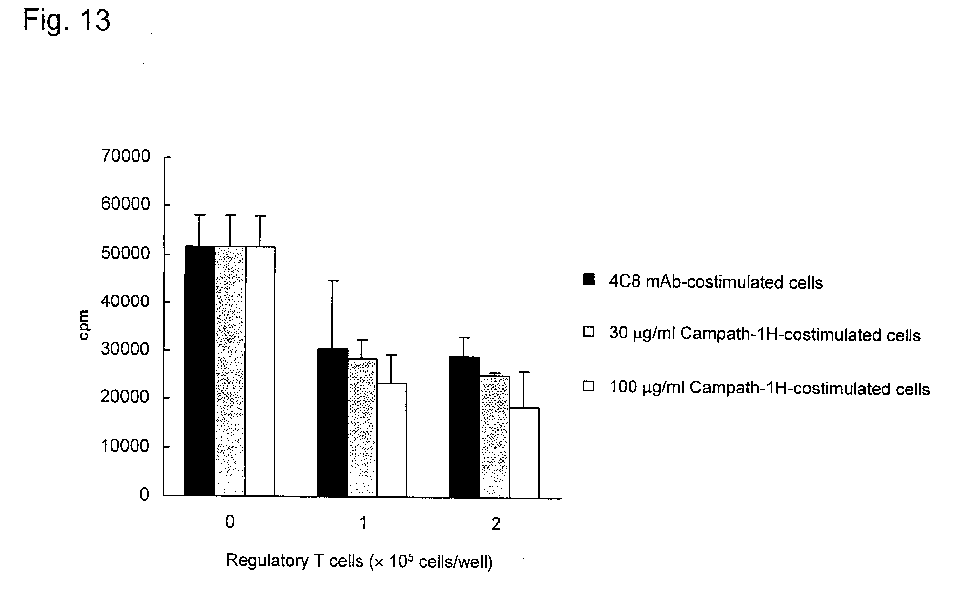

Fig. 13 shows the results of examining the suppressive activity of the Campath-1H-costimulated cells after amplification. The Campath-1H-costimulated cells retained suppressive activity after amplification in a manner similar to the case of the 4C8 mAb-costimulated cells.

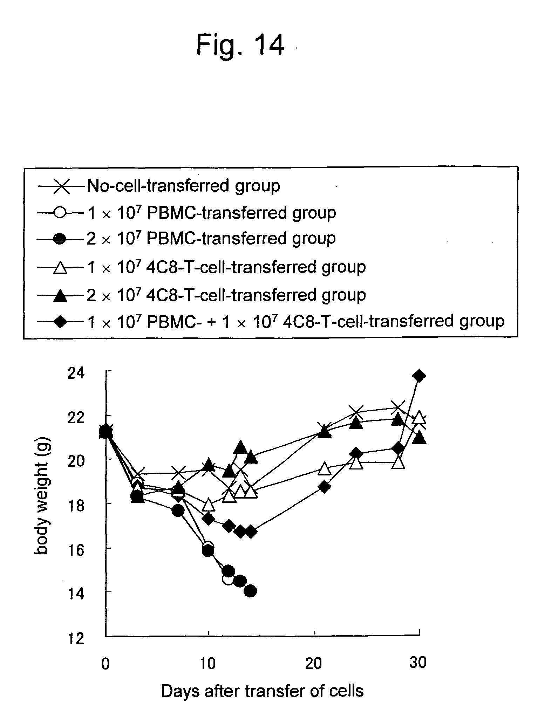

Fig. 14 shows mouse body-weight fluctuations when 1 × 10⁷ or 2 × 10⁷ human PBMCs alone were administered to SCID mice that had been irradiated with a sublethal dose of X-rays on the day following administration of TM-β1 antibodies, 1 × 10⁷ or 2 × 10⁷ regulatory T cells induced by costimulation with 4C8 mAb alone were administered to such SCID mice, or a mixture of 1 × 10⁷ PBMCs and 1×10⁷ regulatory T cells induced by costimulation with 4C8 mAb (total 2×10⁷ cells) was intraperiotoneally administered to such SCID mice.

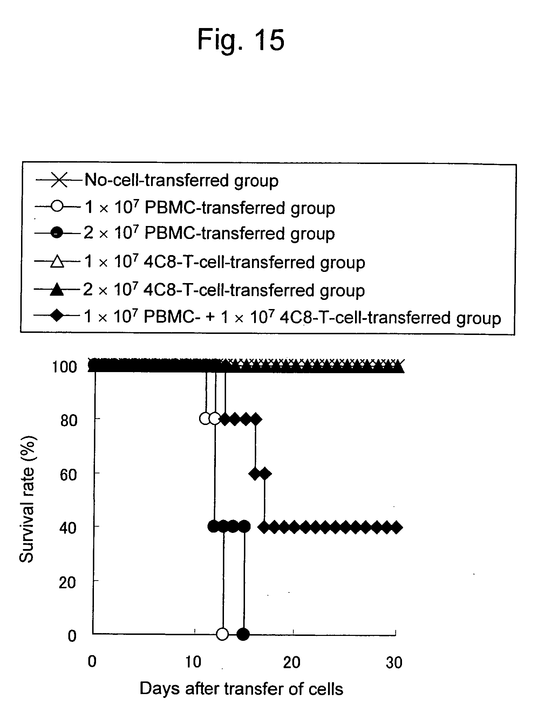

Fig. 15 shows the survival rates of SCID mice when 1 × 10⁷ or 2 × 10⁷ human PBMCs alone were administered to SCID mice that had been irradiated with a sublethal dose of X-rays on the day following administration of TM-β1 antibodies, 1 × 10⁷ or 2 × 10⁷ regulatory T cells induced by costimulation with 4C8 mAb alone were administered to such SCID mice, or a mixture of 1 × 10⁷ PBMCs and 1×10⁷ regulatory T cells induced by costimulation with 4C8 mAb (total 2 × 10⁷ cells) was administered intraperitoneally to such SCID mice.

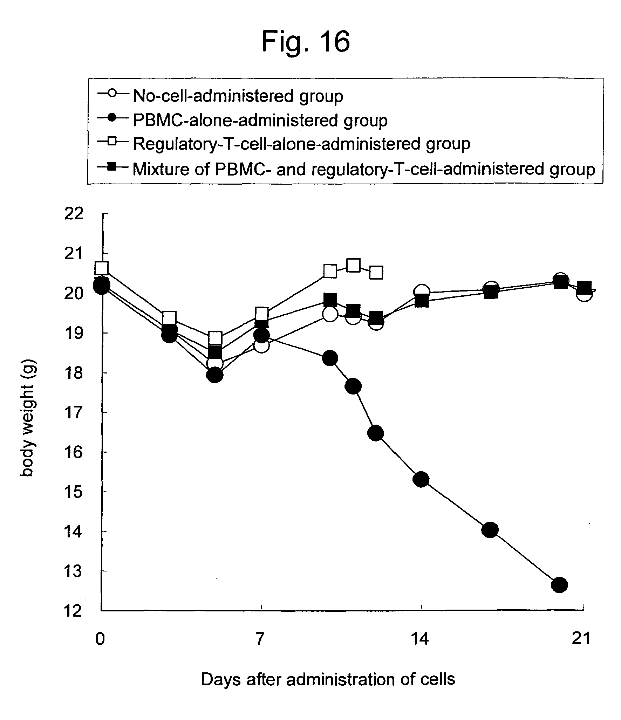

Fig. 16 shows the results of examining mouse body-weight fluctuations when 1.2 × 10⁷ human PBMCs alone were administered to SCID mice that had been irradiated with a sublethal dose of X-rays on the day following administration of TM-β1 antibodies, 1.2 × 10⁷ regulatory T cells induced by costimulation with Campath-1H alone were administered to such SCID mice, or a mixture of 1.2 × 10⁷ PBMCs and 1.2 × 10⁷ regulatory T cells induced by costimulation with Campath-1H (total 2.4 × 10⁷ cells) was intraperitoneally administered to such SCID mice. The mice of the group to which PBMCs alone had been administered showed a trend of transient body weight recovery after approximately 1 week, but then showed continuous body weight reduction. Meanwhile, the mice of the group to which the mixture of PBMCs and the regulatory T cells had been administered showed body weight change similar to those in the group to which no cells had been administered. In addition, since the mice of the group to which the regulatory T cells alone had been administered were autopsied on day 12, body weight was measured until day 12.

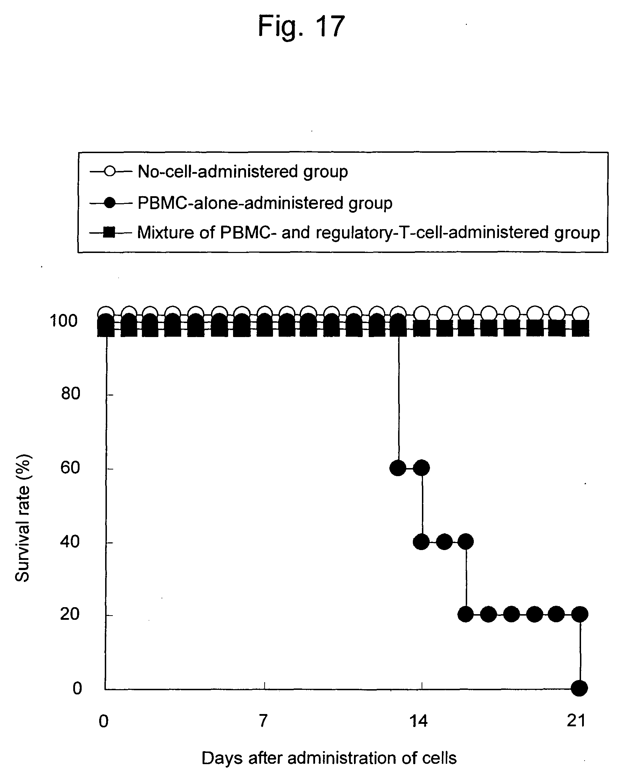

Fig. 17 shows the survival rates of SCID mice, when 1.2 × 10⁷ human PBMCs alone were administered to SCID mice that had been irradiated with a sublethal dose of X-rays on the day following administration of TM-β1 antibodies, 1.2 × 10⁷ regulatory T cells induced by costimulation with Campath-1H alone were administered to such SCID mice, or a mixture of 1.2 × 10⁷ PBMCs and 1.2 × 10⁷ regulatory T cells induced by costimulation with Campath-1H (total 2.4 × 10⁷ cells) was intraperitoneally administered to such SCID mice. All the mice (all the cases) of the group to which PBMCs alone had been administered died by day 21. All the mice (all the cases) of the group to which no cells had been administered and all the mice (all the cases) of the group to which the mixture of PBMCs and the regulatory T cells had been administered survived during the observation period.

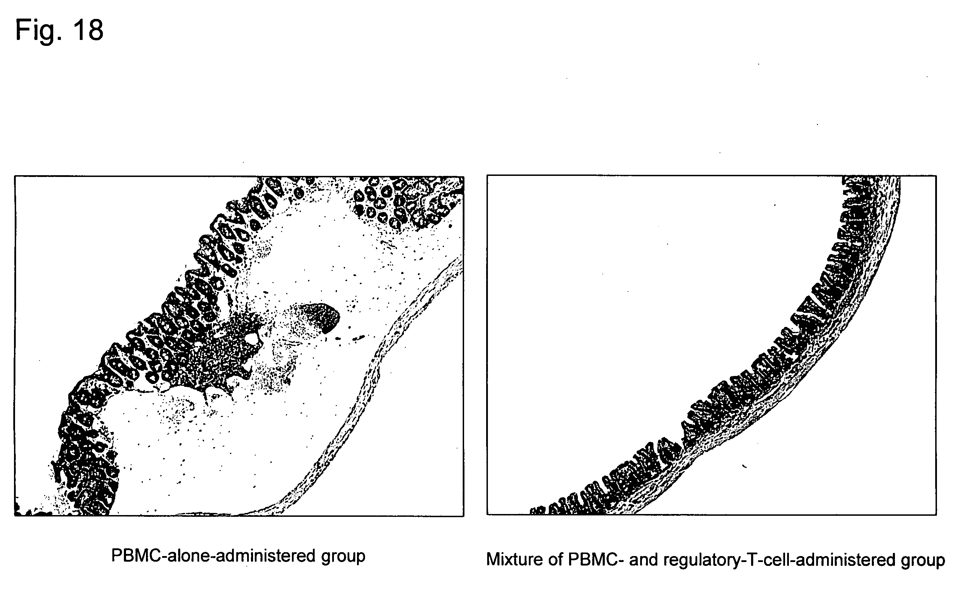

Fig. 18 shows the tissue analysis concerning the digestive tract (cecum) of the mice of the group to which PBMCs alone had been administered and the mice of the group to which the mixture of PBMCs and the regulatory T cells had been administered. Upon observation of (tissue findings concerning) the digestive tracts conducted in the midst of the experimental period, in the case of the group to which PBMCs alone had been administered (left figure), infiltration with mononuclear cells and edema in the submucosal layer, dilatation of blood capillaries, and the like were observed, particularly in the cecum, at various degrees. However, no such changes were observed in the case of the group to which the mixture of PBMCs and the regulatory T cells had been administered (right figure), the group to which regulatory T cells alone had been administered, or the group to which no cells had been administered.

### BEST MODE FOR CARRYING OUT THE INVENTION

The present invention will be described in detail as follows.

In this specification, "CD52 agonist" means a substance that can transmit signals to cells via CD52 by acting on CD52 antigens that are expressed on the surfaces of immunocytes. Responses that can be induced by signals induced by stimulation with the CD52 agonist are: 1) differentiation of the relevant cells into regulatory T cells and/or 2) proliferation of regulatory T cells that retain their properties as regulatory T cells; and/or 3) suppression of transendothelial cell migration of the cells. Examples of such CD52 agonists include natural or synthetic ligands for CD52 antigens, specifically, all molecules that induce signals via CD52 molecules; and antibodies against the CD52 antigens. Such antibodies may be those recognizing any site of CD52, as long as they induce signals via CD52. Examples of antibodies used in the present invention are antibodies having an agonist function for the CD52 antigen. Such antibodies are obtained by selection using the suppression of *in vitro* extravasation of T cells as an index from monoclonal antibodies obtained from animals immunized with human T cells according to the report of Masuyama et al (Masuyama, J. et al., 1999, J. Exp. Med. 189: 979-989; International Patent Publication WO99/12972). Moreover, anti-CD52 antibody fragments retaining antigen-recognizing sites of antibody molecules are also useful as the CD52 agonists of the present invention.

Furthermore, another example of such a CD52 agonist useful in the present invention is a rat humanized antibody Campath-1H (Alemtuzumab). This antibody is marketed and available as Campath (trademark). The detailed method for producing this antibody is disclosed in JP Patent Publication (Kohyo) No. 2-503514 A (1990).

Desired antibodies can be efficiently obtained using as T cells (to be used as antigens) mononuclear cells that pass through and migrate out the monolayers after co-culture of a peripheral blood mononuclear cell fraction collected from a human with human umbilical vein endothelial cells (HUVEC) cultured on collagen gel to form monolayers. To select hybridomas, an anti-4C8 antibody that is produced by a hybridoma JM-1 (deposited with the International Patent Organism Depositary, the National Institute of Advanced Industrial Science and Technology (AIST) under accession number FERM BP-7757 on September 26, 2001) is used as a competitive reagent. The use of such a reagent is combined with selection using suppression of T-cell staining with antibodies to be tested (that is, recognition of the same epitope as that of the anti-CD52 antibody produced by the hybridoma JM-1) as an index. Thus, the obtainment of desired antibodies is further facilitated. Through a combination of a step for selecting antibodies that recognize the same epitope as that recognized by the anti-4C8 antibody and a step for evaluating ability of inducing differentiation and/or proliferation of regulatory T cells, which is described in the examples of the present invention, the antibodies used in the present invention can be easily obtained. Furthermore, for example, through transplantation of variable regions of the anti-CD52 antibodies produced by the hybridoma JM-1 into the frameworks of human antibodies, so-called humanized antibodies can also be obtained as antibodies used in the present invention. Furthermore, through the use of mice (e.g., Tomizuka et al., 2000, Proc. Natl. Acad. Sci. U.S.A., 97: 722) retaining unrearranged human antibody genes and producing human antibodies specific to antigens by sensitization with the antigens, human antibodies as antibodies to be used in the present invention can be obtained.

In the method of the present invention, CD52 can exert its ability to induce differentiation and to promote proliferation of regulatory T cells by causing the CD3 agonist, in addition to CD52, to act on immunocytes expressing CD52. A CD3-molecule-mediated signal transduction system, which is a major stimulation-transmitting pathway involved in T cell differentiation, is considered to be a primary stimulation pathway. Thus, a CD52-mediated pathway is so-called a costimulation pathway. In this specification, provision of CD52-mediated stimulation in addition to CD3-molecule-mediated stimulation may be referred to as costimulation with CD52. In this specification, "CD3 agonist" means a substance that can act on the CD3 molecules expressed on the surfaces of immunocytes so as to cause CD3-mediated signal transduction into the cells and to cause a reaction to take place, by which differentiation of the relevant cells is promoted. Examples of such CD3 agonists include agonistic anti-CD3 antibodies such as OKT3 (ATCC CRL-8001), UCHT1 (B. D. PharMingen), and HIT3a (B. D. PharMingen). Moreover, agonists for various T cell antigen receptors, and particularly, antibodies having agonist action or fragments thereof, can also be used as the CD3 agonists in the present invention, because they cause the formation of complexes of T cell antigen receptors and CD3 and cause CD3-mediated signal transduction into cells. Specifically, an example of such an agonist is an OT145 antibody (Posnett et al., 1986, Proc. Natl. Acad. Sci. U.S.A., 83 (20): 7888-92) against a human T cell antigen receptor V beta 6.7. Furthermore, substances that can also be used herein have agonist action as a result of recognition by T-cell antigen receptors, such as soluble HLA molecules, or tetramer molecules of HLA molecules and antigen peptides.

Migration of inflammatory cells such as T cells and macrophages through vascular endothelia to extravascular inflammatory sites is an important step in inflammatory reactions. Many attempts have been conducted to control allergies or various immune diseases by inhibiting the action of molecules such as chemokines or adhesion factors that are known to be involved in this step using low-molecular-weight antagonists, antibodies, or the like (Yang, G.X. et al., (2003) Med. Res. Rev. 23: 369-392; Aydt, E. et al., (2002-2003) 70: 297-301; Erin, E. M. et al., (2002) Curr. Drug Targets Inflamm. Allergy 1: 201-214; Saeki, T. et al., (2003) Curr. Pham. Des. 9: 1201-1208). Now it has been newly revealed that CD52 is involved in transendothelial cell migration of T cells. Thus, the possibility is revealed that extravasation of T cells is inhibited by administration of an antibody or a ligand against CD52 or a low-molecular-weight agonist or an antagonist, thereby suppressing inflammatory reaction and/or immune reaction.

Regarding Campath-1H (Alemtuzumab), the anti-CD52 antibody, clinical studies using the antibody for suppressing immunity have already been conducted regarding articular rheumatism, multiple sclerosis, kidney-transplantation rejection, and the like (Isaacs, J. D. et al., 2001, Arthritis Rheum. 44: 1998-2008; Coles, A. J. et al., (1999) Lancet 354: 1691-1695; Calne, R. et al., (1999) Transplantation 68: 1613-1616). The action mechanism of Campath-1H involves the removal of CD52-expressing lymphocytes due to cytotoxicity of complements or antibody-dependent cellular cytotoxicity (Hale, G., 2001, Cytotherapy, 3:137-143). Removal of lymphocytes results in a state of reduced number of lymphocytes until new lymphocytes are generated again by differentiation. A clinical test of Campath-1H on articular rheumatism has revealed that such a state of reduced number of lymphocytes induced by Campath-1H persists for a very long time. Times required for various lymphocytes that had been reduced in number after administration of Campath-1H to recover normal cell counts are 1 month in the case of NK cells or monocytes and 3 to 6 months in the case of B cells. In the case of T cells, the cell count of T cells remained at a level lower than the normal level even after 3 years (Isaacs, J.D. et al., 2001, Arthritis Rheum. 44: 1998-2008). Specifically, even after treatment with Campath-1H has ceased, a side effect is generated such that a high-risk state of infection or the like is continued. To solve this problem, newly obtained findings have revealed that anti-CD52 antibodies not having cell-removing action are expected to have immunosuppressive action. Through administration of such anti-CD52 antibodies having suppression of transendothelial cell migration as an action mechanism and not having the lymphocyte-removing effect, an immunosuppressive effect can be obtained without causing a persistently reduced number of lymphocytes, unlike the case of Campath-1H.

Such anti-CD52 antibodies not having the lymphocyte-removing effect can be selected by, for example, the following method. First, it is known that both cytotoxicity of complements, which is a major action mechanism for cell removal by antibodies, and antibody-dependent cellular cytotoxicity greatly depend on antibody subclasses. For example, IgG4 among human antibodies and IgG1 among mouse antibodies are of subclasses characterized by low cytotoxicity of complements and low antibody-dependent cellular cytotoxicity (Maloney, D. G. (1998) "Unconjugated monoclonal antibody therapy of lymphoma" in: Grossbard ML, ed. *Monoclonal antibody-based therapy of cancer,* New York: Dekker: 53-79). Antibodies of such subclasses not having cell-removing activity are selected or the Fc portions of such subclasses are introduced and/or substituted by gene recombination techniques and then *in vitro* screening is carried out for cytotoxic activity of the antibodies. Cytotoxic activity of complements can be screened for by the following method. Target cells expressing CD52 are incubated with ⁵¹Cr at 37°C for 1 hour, thereby labeling the target cells with ⁵¹Cr. The cells are washed and then inoculated on 96-well plates. The anti-CD52 antibodies and human complements are added to the plates, followed by incubation at 37°C for 2 hours. After incubation, supernatants are collected, and then the count of ⁵¹Cr released in the supernatants is measured using a Top count (Packard) or the like. Thus, cytotoxic activity of the complements of the antibodies can be evaluated. Moreover, antibody-dependent cellular cytotoxic activity can be screened for by the following method. Target cells expressing CD52 are incubated with ⁵¹Cr at 37°C for 1 hour, thereby labeling the target cells with ⁵¹Cr. The cells are washed and then inoculated on 96-well plates. The anti-CD52 antibodies and human peripheral blood mononuclear cells are added to the plates, followed by incubation at 37°C for 4 hours. After incubation, supernatants are collected, and then the count of ⁵¹Cr released in the supernatants is measured using a Top count or the like. Thus, antibody-dependent cellular cytotoxic activity of the antibodies can be evaluated. That the antibodies selected by the above method have no lymphocyte-removing activity can be confirmed by finally monitoring the number of lymphocytes upon administration to a human.

As described above, transendothelial cell migration is suppressed by causing the CD52 agonist to act on CD52, so that immune responses can be suppressed. Furthermore, differentiation induction of regulatory T cells is promoted and/or proliferation of regulatory T cells is promoted by co-stimulation of with CD3 and CD52. Simultaneous stimulation with CD3 and CD52 also provides suppression of immune responses. Hence, a pharmaceutical composition containing as an active ingredient the CD52 agonist and a pharmaceutical composition containing as active ingredients the CD52 agonist and the CD3 agonist, which are provided by the present invention, can be useful as immunosuppressive agents. Furthermore, such pharmaceutical compositions can be useful as drugs targeting the action mechanisms of specific immune systems, and specifically, as agents for inducing differentiation and/or promoting proliferation of regulatory T cells.

Furthermore, the pharmaceutical compositions provided by the present invention may be administered *in vivo* or may be used for treating *ex vivo* materials collected from patients or other humans including immunocytes and particularly T cells or peripheral blood containing immunocytes, lymph fluid, lymph node cells, and thymus cells. The pharmaceutical compositions of the present invention can be formulated by known methods. Specifically, a pharmaceutical preparation is produced, to which various additives that are acceptable in terms of therapeutic effects, such as a carrier, a pH buffer agent, a stabilizer, and an excipient are added. Such a preparation preferably contains a physiologically acceptable diluent or a carrier. Examples of an appropriate carrier include, but are not limited to, a physiological saline solution, a phosphate buffered saline solution, a phosphate buffered saline glucose solution, and a buffered physiological saline solution. Alternatively, the CD52 agonist is freeze-dried, and then used when necessary by adding the above buffered aqueous solution for reconstruction. Examples of the route of administration include an oral route using administration forms of the above preparations, such as tablets, capsules, granules, powders, and syrup pharmaceuticals, and a parenteral route using administration forms of the same, such as injections, drops, and suppositories.

Administration methods and doses of the above pharmaceutical compositions can be appropriately determined in the processes of preclinical tests and clinical tests. For example, the dose in the case of oral administration differs depending on symptoms, age, body weight, and the like and is generally approximately 0.01 mg to 1000 mg per day for an adult. The dose can be administered once or several separate times. Furthermore, in the case of parenteral administration, a single dose of approximately between 0.01 mg and 1000 mg can be administered by subcutaneous injection, intramuscular injection, or intravenous injection.

Disease subjected to treatment is disease that requires procedures providing an immunosuppressive effect. Specific examples of such procedures include procedures undertaken before or after transplantation of organs or cells for the purpose of treating or preventing graft versus host disease (GvHD) or graft rejection and procedures undertaken for treating or preventing autoimmune disease such as rheumatism or allergic disease such as contact hypersensitivity.

A therapy that can be employed in the present invention comprises treating *ex vivo* materials collected from patients or other humans including immunocytes or peripheral blood containing immunocytes, lymph fluid, lymph node cells, or thymus cells so as to proliferate regulatory T cells and then returning the cells into patients' bodies. A stem cell transplantation therapy, which comprises collecting peripheral blood or marrow cells from living bodies, and then returning the cells into the patients' bodies has already been implemented. Furthermore, a cancer therapy that comprises artificially treating dendritic cells that constitute a type of immunocytes and then returning the cells into the patients' bodies has been conducted (M. Jefford, et al., The Lancet Oncology, 2: 343-353, June, 2001). By causing the CD52 agonists and the CD3 agonists to act on the collected immunocytes, differentiation induction and/or proliferation promotion for regulatory T cells can be caused. Then, the regulatory T cells are proliferated and then the cells are returned into the patients' bodies, so that a therapeutic or preventive effect can be exerted. Implementation of such so-called *ex vivo* methods can be said to constitute almost direct reproduction of experimental systems at therapeutic sites, where such systems have been developed at basic research sites. Such *ex vivo* methods can be said to be methods with lower risks for practical application compared with cases where the *in vivo* administration of a drug may not result in such drug exerting its therapeutic effects as expected, as a result of effects of *in vivo* absorption, metabolism, interferential action due to unknown factors, or the like.

Moreover, the present invention also encompasses, for developing a drug having effects of inducing differentiation and/or promoting proliferation of regulatory T cells and/or effects of suppressing transendothelial cell migration of immunocytes, causing cells expressing CD52 to come into contact with a candidate compound, detecting the interaction between the two or responses of CD52 to stimulation, and thus screening for a compound that can be a CD52 agonist using interaction with CD52 as an index.

### Example 1: Identification of 4C8 antigen

We have already revealed that the 4C8 monoclonal antibody against the 4C8 antigen (where the 4C8 antigen is a membrane protein expressed by some immune system cells and was discovered for the first time during identification of a molecule involved in transendothelial migration of human T cells after their adhesion to vascular endothelial cells) suppresses *in vitro* extravasation of T cells (Masuyama, J. et al., 1999. J. Exp. Med. 189: 979-989; International Patent Publication WO99/12972).

First, identification of the 4C8 antigen was attempted. The 4C8 antigen was isolated and identified by constructing a cDNA library using CD3-positive cells as a gene source in the form of a 4C8 mAb-positive cell fraction and then concentrating COS-1 cells that had been caused to transiently express the library using 4C8 mAb and MACS system (Miltenyi Biotec).

Peripheral blood mononuclear cells were separated from heparinized human peripheral blood by density gradient centrifugation using Ficoll-Hypaque. CD3-positive T cells were prepared using anti-CD3 antibodies (Miltenyi Biotec) and MACS system. Total RNA, 90 µg, was obtained from 10⁸ CD3-positive T cells using RNeasy Mini Kit (QIAGEN Inc). Poly (A) ⁺RNA was purified using Oligotex™-dT30 <Super> mRNA Purification Kit (TAKARA BIO Inc.). Poly (A) ⁺RNA, 1.7 µg, was obtained from 90 µg of the total RNA. cDNA synthesized from the poly (A) ⁺RNA using Superscript™Choice System for cDNA Synthesis (Invitrogen Co.) was subjected to size fractionation by agarose gel electrophoresis. One-Kb to ten-Kb cDNAs were cloned into eukaryotic cell expression vector pEF18S (Ohashi, H. et al., 1994, Proc. Natl. Acad. Sci. U.S.A. 91: 158-162). As a result, a CD3-positive-T-cell-derived cDNA library consisting of 7×10⁶ independent clones was obtained.

Plasmids, 270 µg, was obtained from 500 ml of LB (Luria-Bertani) culture solution of the above library using Endofree Plasmid Maxi Kit (QIAGEN Inc.). The plasmids, 100 µg, was transiently introduced into 2×10⁷ COS-1 cells using TransIT-LT1 (TAKARA BIO Inc.). 4C8 mAb-positive cells were purified from COS-1 cells that had been caused to transiently express the library using the antibody and MACS system. Plasmids were collected from the 4C8 mAb-positive COS-1 cells by Hirt method (Hirt B., 1967, J. Mol. Biol., 26: 365-369). The plasmids were introduced into *Escherichia coli* (ElectroMAX DH10B, Invitrogen Co.) for amplification and then prepared in a manner similar to the above. The above process ranging from introduction into COS-1 cells to collection of plasmids was further repeated 3 times.

Independent plasmid clones, 384 clones, were randomly isolated from the thus obtained concentrated library. The plasmid clones were transiently introduced into COS-1 cells in a manner similar to the above and then 4C8-positive clones were distinguished from other clones using a FACSCalibur (Becton-Dickinson). As a result, it was revealed that 3 clones were 4C8-positive (Fig. 1).

The nucleotide sequences of the positive clones were determined using ABI PRISM 3700 DNA Analyzer (Applied Biosystems), followed by BLAST searches of nucleotide databases. All of the 3 positive clones were revealed to encode CD52 (Xia, M. Q. et al., 1991, Eur. J. Immunol. 21: 1677-1684). Products resulting from expression of the cDNA by COS-1 cells were also stained with Campath-1H, which is known to be an anti-CD52 antibody. When Stainability with 4C8 mAb of cells transfected cDNAs of CD48, CD58 and CD59, which are GPI anchor proteins like CD52, was analyzed by FACS Calibur, the results were negative. Accordingly, it was confirmed that 4C8 mAb does not recognize the common structure of GPI anchor proteins (not shown in the figures). Therefore, 4C8 was concluded to be CD52.

### Example 2: Induction of regulatory T cell by costimulation with Campath-1H

Examination was carried out concerning whether regulatory T cells are induced from CD4-positive T cells in a manner similar to that in the case of costimulation using 4C8 mAb, when a monoclonal antibody Campath-1H (C1H) whose antigen is CD52 is used for costimulation.

Peripheral blood mononuclear cells were separated by a density gradient centrifugation method using Ficoll-Paque Plus (Amersham Pharmacia) from the peripheral blood of a healthy human adult volunteer. CD4-positive T cells were prepared from peripheral blood mononuclear cells by negative selection according to the operational procedures of a reagent manufacturer using MACS CD4+ T Cell Isolation Kit (Miltenyi Biotec).

Antibodies were immobilized onto plates as described below. The anti-CD3 antibodies (ORTHOCLONE OKT3, Ortho Biotech) prepared to a concentration of 100 ng/ml using PBS were dispensed on 48-well plates (Costar) and incubated at 4°C for 24 hours for immobilization, and 4C8 mAb or Campath-1H (CAMPATH, Berlex) prepared to a concentration of 10 µg/ml using PBS was similarly immobilized.

CD4-positive T cells prepared by the above method were suspended in RPMI1640 medium (GIBCO) supplemented with 10% FCS and 15mM HEPES buffer. The resultant was inoculated on the plates having antibodies immobilized thereon by the above method at 8×10⁵ cells/well, followed by culture at 37°C and 5% CO₂ for 3 days in CO₂ incubator. After culture, the cells were collected and then washed. The cells were then re-suspended in media and then inoculated on 24-well plates (Costar) at 1×10⁶ cells/well. After another 4 days of culture without stimulation so as to cause the cells to be in a resting state, the 4C8 mAb-costimulated cells or the Campath-1H-costimulated cells were subjected to suppression assay.

Suppression assay was carried out as follows. CD4-positive T cells and peripheral blood mononuclear cells irradiated with X-rays (5000 rad) were inoculated together on 96-well U-bottomed plates (ICN) at 1 × 10⁵ cells/well and 4 × 10⁵ cells/well, respectively. Anti-CD3 antibodies were added at a final concentration of 25 ng/ml, followed by 3 days of culture. Thereafter, a control group to which no regulatory T cells had been added and a group to which the 4C8 mAb-costimulated cells or the Campath-1H-costimulated cells (both of which were irradiated with X-rays (5000 rad) as regulatory T cells) had been added at 0.5 × 10⁵ to 2 × 10⁵ cells/well were compared concerning [³H] thymidine incorporation, thereby evaluating suppressive activity. [³H] thymidine incorporation was evaluated by adding [³H] thymidine at 0.2 µCi/well on day 3 of culture, collecting the cells after 8 hours, and then measuring the count of [³H] thymidine incorporated by the cells using a beta plate (Perkin Elmer). All the cells used in the first assay were derived from the same donor.

As shown in Fig. 2, the Campath-1H-costimulated cells suppressed the proliferation of CD4-positive T cells due to stimulation with anti-CD3 antibodies in a cell number dependent manner, similarly to the case of the 4C8 mAb-costimulated cells.

### Example 3: Suppression of transendothelial cell migration of CD3-positive T cell by Campath-1H

Examination was carried out using Campath-1H concerning whether suppressive activity of the transendothelial cell migration of CD3-positive T cells that was reported in the case of 4C8 mAb (Masuyama, J. et al., 1999, Journal of Experimental Medicine, 189(6), 979-989) is also observed by the use of another type of anti-CD52 antibody. The method therefor was conducted as described below based on the method reported in the above paper.

CD3-positive T cells were prepared by concentrating CD3-positive T cell fraction from peripheral blood mononuclear cells using a nylon wool column and then performing negative selection using anti-CD16-magnetic antibodies (Advanced Magnetics, Inc.).

Human umbilical vein endothelial cells were cultured to be confluent on 96-well flat-bottomed plates (Becton Dickinson) having collagen gel disposed at 50 µl/well therein and then subjected to assay. CD3-positive T cells were pre-treated by adding 0.3 µg/ml to 3 µg/ml 4C8 mAb or Campath-1H to M199 medium (GIBCO) supplemented with 0.5% BSA and then incubated on ice for 20 minutes. The CD3-positive T cells (3 × 10⁵/well) were treated with antibodies and then inoculated without washing on the above plates having human umbilical vein endothelial cells disposed therein. The plates were centrifuged at 50 × g for 1 minute, followed by incubation at 37°C for 3 hours. Unadhered CD3-positive T cells and vascular endothelial cells were removed by washing with EDTA. The number of T cells that had migrated into the collagen gel under the layers of human umbilical vein endothelial cells was counted using a phase contrast microscope. A group treated with the antibodies and an untreated group were compared for the number of cells that had migrated per unit area. All the experiments were conducted in triplicate.

As shown in Fig. 3, Campath-1H suppressed transendothelial cell migration of CD3-positive T cells in a manner similar to the case of 4C8 mAb. Hence, it was revealed that Campath-1H, the anti-CD52 antibody, induces suppression of transendothelial cell migration in a manner similar to the case of 4C8 mAb.

### Example 4-1: Suppression of CD4-positive-T-cell-mixed lymphocyte culture reaction by regulatory T cell induced by costimulation with anti-CD52 antibody

CD4-positive-T-cell-mixed lymphocyte culture reaction assay was carried out to examine whether regulatory T cells induced by costimulation with anti-CD52 antibodies suppress the reaction of CD4-positive T cells due to alloantigen stimulation. 4C8 mAbs were used as anti-CD52 antibodies.

Regulatory T cells were induced by the method described in Example 2. Monocyte-derived mature dendritic cells used as stimulator cells were induced as follows. CD14-positive cells were prepared from peripheral blood mononuclear cells by positive selection according to the operational procedures of a reagent manufacturer using MACS microbeads CD14 (Miltenyi Biotec). The CD14-positive cells were suspended in RPMI1640 medium supplemented with 10% FCS, 2-mercaptoethanol (GIBCO), 100ng/ml IL-4, and 50ng/ml GM-CSF and then inoculated on 6-well plates (Falcon) at 3 × 10⁶ cells/well. After 5 days of culture, LPS with a final concentration of 10 ng/ml was added and then the resultant was further cultured for 24 hours, thereby inducing monocyte-derived mature dendritic cells. Portion of the cells were subjected to analysis using a flow cytometer (FACScan, Becton Dickinson), thereby confirming the expression of mature dendritic cell markers.

The mixed lymphocyte culture reaction was conducted by inoculating CD4-positive T cells (1×10⁵ cells/well) prepared from donor A and monocyte-derived mature dendritic cells (1 × 10⁴cells/well) prepared from a different donor, donor B, on 96-well U-bottomed plates, followed by 4 days of culture. Thereafter, a control group to which no regulatory T cells had been added and a group to which the 4C8 mAb-costimulated cells (where the cells were irradiated with X-rays (5000 rad) as regulatory T cells) had been added at 1 × 10⁵ to 2 × 10⁵ cells/well were compared concerning [³H] thymidine incorporation, thereby evaluating suppressive activity. [³H] thymidine incorporation was measured in a manner similar to Example 1, except culture time from addition of [³H] thymidine to the collection of cells was determined to be 16 hours.

As shown in Fig. 4, the 4C8 mAb-costimulated cells suppressed the CD4-positive-T-cell-mixed lymphocyte culture reaction in a cell number dependent manner.

### Example 4-2: Suppression of CD4-positive-T-cell-mixed-lymphocyte culture reaction by regulatory T cell induced by costimulation with Campath-1H

Examination was carried out concerning whether the reaction of CD4-positive T cells due to alloantigen stimulation is also suppressed by regulatory T cells induced by the use of Campath-1H as an anti-CD52 antibody. The experiment was conducted by a method similar to that in the above Example 4-1, except the reaction period was determined to be 3 days.

As shown in Fig. 5, the Campath-1H-costimulated cells suppressed the CD4-positive-T-cell-mixed lymphocyte culture reaction in a cell number dependent manner.

Hence, it was shown that regulatory T cells induced by costimulation with Campath-1H suppress the reaction of CD4-positive T cells due to alloantigen stimulation in a manner similar to the case of regulatory T cells induced by costimulation with 4C8 mAb.

### Example 5-1: Suppression of CD8-positive-T-cell-mixed-lymphocyte culture reaction by regulatory T cell induced by costimulation with anti-CD52 antibody

To examine whether regulatory T cells induced by costimulation with anti-CD52 antibodies also show suppression of the proliferation of CD8-positive T cells due to alloantigen stimulation, CD8-positive-T-cell-mixed lymphocyte culture reaction assay was carried out. 4C8 mAb was used as an anti-CD52 antibody.

CD8-positive T cells were prepared from peripheral blood mononuclear cells by negative selection according to the operational procedures of a reagent manufacturer using MACS CD8+ T Cell Isolation Kit (Miltenyi Biotec). Regulatory T cells were induced by the method described in Example 2. Monocyte-derived mature dendritic cells used as stimulator cells were induced in a manner similar to Example 4-1.

The mixed lymphocyte culture reaction was conducted by inoculating CD8-positive T cells (1×10⁵ cells/well) prepared from donor A and monocyte-derived mature dendritic cells (1×10⁴ cells/well) prepared from a different donor, donor B, on 96-well U-bottomed plates, followed by 3 days of culture. Thereafter, a control group to which no regulatory T cells had been added and a group to which the 4C8 mAb-costimulated cells (where the cells were irradiated with X-rays (5000 rad) as regulatory T cells) had been added at 1 × 10⁵ to 2 × 10⁵ cells/well were compared for [³H] thymidine incorporation, thereby evaluating suppressive activity. [³H] thymidine incorporation was measured in a manner similar to Example 4-2.

As shown in Fig. 6, the 4C8 mAb-costimulated cells suppressed the CD8-positive-T-cell-mixed lymphocyte culture reaction in a cell number dependent manner.

### Example 5-2: Suppression of CD8-positive-T-cell-mixed lymphocyte culture reaction by regulatory T cell induced by costimulation with Campath-1H

CD8-positive-T-cell-mixed lymphocyte culture proliferation assay was carried out to examine whether regulatory T cells induced using Campath-1H as an anti-CD52 antibody also show suppression against the reaction of CD8-positive T cells due to alloantigen stimulation. The experiment was conducted using the method similar to that in Example 5-1.

As shown in Fig. 7, the Campath-1H-costimulated cells suppressed the mixed lymphocyte culture reaction in a cell number dependent manner.

Hence, it was shown that regulatory T cells induced by costimulation with Campath-1H suppress the proliferation of the CD8-positive T cells due to alloantigen stimulation in a manner similar to the case of regulatory T cells induced by costimulation with 4C8 mAb.

### Example 6: Induction of regulatory T cell from CD4-positive T cell after alloantigen reaction

Examination was carried out concerning whether regulatory T cells having antigen-selective suppressive activity can be induced by applying costimulation with anti-CD52 antibodies to CD4-positive T cells after alloantigen reaction.

Monocyte-derived mature dendritic cells used as stimulator cells were induced as follows. CD14-positive cells prepared using the method shown in Example 4-1 were suspended in X-VIVO-15 medium (Cambrex) supplemented with 1% heat-inactivated autoplasma, 100ng/ml IL-4, and 50ng/ml GM-CSF. After 7 days of culture at 3×10⁶ cells/well on 6-well plates, the cells were collected and then washed. The cells were then suspended in media prepared by adding 10 ng/ml IL-1β (R&D systems), 3 µg/ml IL-6 (produced in the facilities of KIRIN BEER KABUSHIKI KAISHA), 10 ng/ml TNFα (PeproTech), and 1 µg/ml prostaglandin E2 (Sigma) to the above media. The resulting cells were inoculated at 1×10⁶ cells/well onto 6-well plates, and then further cultured for 2 days, thereby inducing monocyte-derived mature dendritic cells. CD4-positive CD45RA-positive T cells were prepared from CD4-positive T cells (isolated by the method described in Example 2 above) by positive selection according to the operational procedures of a reagent manufacturer using MACS CD45RA MicroBeads (Miltenyi Biotec).

Alloantigen reaction and induction of regulatory T cells were carried out as follows. CD4-positive CD45RA-positive T cells (1×10⁶cells/well) prepared from donor A and monocyte-derived mature dendritic cells (1×10⁵ cells/well) prepared from a different donor, donor B, were suspended in X-VIVO-15 media supplemented with pyrubic acid (Gibco) and MEM non-essential amino acid solutions (Gibco), and then inoculated on 24-well plates. After 6 days of culture, the cells were collected, re-prepared to a concentration of 1×10⁶ cells/well, and then further cultured for 4 days. The resulting cells were used as post-alloantigen-reaction T cells. Specifically, regulatory T cells were induced from the cell population. Regulatory T cells were induced by the method shown in Example 2, except post-allo-reaction T cells were used instead of CD4-positive T cells. As a control group, regulatory T cells were induced from CD4-positive T cells prepared from donor A. Hereinafter, regulatory T cells induced from post-allo-reaction T cells are referred to as post-alloantigen-reaction regulatory T cells. Regulatory T cells induced from CD4-positive T cells are referred to as control regulatory T cells.

The suppressive activity of each type of regulatory T cell was evaluated by the mixed lymphocyte culture reaction described in Example 4. However, as stimulator cells, monocyte-derived mature dendritic cells prepared from donor B (used upon the preparation of the post-alloantigen-reaction T cells) or monocyte-derived mature dendritic cells prepared from a third subject, donor C, were used. X-VIVO-15 medium supplemented with pyrubic acid and non-essential amino acid slution were used for the reaction. The culture period was determined to be 3 days.

As shown in Fig. 8, in the reaction to the cells derived from donor B, post-alloantigen-reaction regulatory T cells showed suppressive activity that was stronger than that of control regulatory T cells. However, in the reaction against the third subject, donor C, both suppressive activities were equivalent to each other. As described above, it was revealed that regulatory T cells showing alloantigen-selective suppressive activity are obtained by inducing regulatory T cells using anti-CD52 antibodies from the post-alloantigen-reaction T cells.

### Example 7-1: In vitro amplification of regulatory T cell induced by costimulation with anti-CD52 antibody

Examination was carried out concerning whether amplification is possible by culturing regulatory T cells induced by costimulation with anti-CD52 antibodies in the presence of IL-2.

CD4-positive T cells were inoculated at 8×10⁵ cells/well onto plates on which anti-CD3 antibodies and 4C8 mAbs had been immobilized by the method described in Example 2 and then cultured for 3 days. After being collected and washed following culture, the cells were re-suspended in media containing 100 U/ml IL-2 (Chiron) and then inoculated on 24-well plates at 1×10⁶ cells/well. After 2 days of culture, the cells were collected and then re-prepared to a concentration of 1×10⁶ cells/well in media containing 100 U/ml IL-2, followed by 3 days of culture. After total of 8 days of culture, the cells were collected. The first suppression assay (day 8) was carried out by the method described in Example 2 using portion of the cells. Furthermore, some of the cells after 8 days of culture were washed, cultured on plates upon which anti-CD3 antibodies and 4C8 mAbs had been immobilized for 3 days, and then cultured for 4 days without stimulation by the method shown in Example 2. The second suppression assay (day 15) was carried out using the thus obtained cells. As a control group, cells subjected to completely the same treatment except for having been cultured in media not containing IL-2 during a period from day 3 to day 8 of culture were used for each suppression assay.

As shown in Fig. 9, the number of cells had increased 10-fold or more after 5 days of culture in the presence of IL-2. However, as shown in Fig. 10, in the first suppression assay (day 8) carried out immediately after culture in the presence of IL-2, suppressive activity was significantly attenuated compared with the case of the control group. However, as shown in Fig. 11, in the second suppression assay (day 15) wherein the cells were subjected again to costimulation with anti-CD52 antibodies, recovery of suppressive activity was observed. The number of the cells had increased 30-fold after the whole culture period of 15 days.

### Example 7-2: In vitro amplification of regulatory T cell induced by costimulation with Campath-1H

Examination was carried out concerning whether amplification is also possible by culturing regulatory T cells induced using Campath-1H as an anti-CD52 antibody in the presence of IL-2.

Regulatory T cells were induced and amplified by the method described in Example 7-1, except Campath-1H (immobilized on plates at 30 µg/ml or 100 µg/ml) was used for costimulation. Furthermore, culture in the presence of IL-2 was carried out for 4 days in this example, and suppression assay was carried out only on day 14. A group to which IL-2 had not been added was not provided. A group for which 4C8 mAb had been used for costimulation, similarly to Example 7, wasdetermined to be a control group. Campath-1H tended to have somewhat lower activation ability than 4C8 mAb. Hence, to obtain sufficient activation, the concentration in this example was determined to be as high as 30 µg/ml or 100 µg/ml as a condition for immobilization.

As shown in Fig. 12, by culture in the presence of IL-2, the Campath-1H-costimulated cells were amplified 10-fold or more in a manner similar to the case of the 4C8 mAb-costimulated cells. Moreover, as shown in Fig. 13, the Campath-1H-costimulated cells retained suppressive activity after amplification, in a manner similar to the case of 4C8 mAb-costimulated cells.

Therefore, it was shown that regulatory T cells retaining suppressive activity can be amplified by repetition of costimulation with anti-CD52 antibodies after amplification in the presence of IL-2.

### Example 8-1: Suppression of lethality against mouse due to transfer of human peripheral blood into SCID mouse by simultaneous transfer of regulatory T cell and in vivo safety of regulatory T cell

To suppress the activity of NK cells so as to facilitate the adhesion and integration of human cells in SCID mice (6-week-old, male, obtained from CLEA Japan, Inc.,), TM-β1 antibodies (20 µg/mouse, Pharmingen) that recognize IL-2 receptor β chains were intraperitoneally administered on the day before the transfer of human cells, and then the mice were irradiated with a sublethal dose (2.5 Gy) of X-rays on the day of the transfer of the human cells. The human cells were then transferred. Mononuclear cells (PBMC) were obtained by subjecting leukapheresis sample from a healthy adult to specific gravity centrifugation using Lymphoprep (AXIS-SHIELD). Alternatively, regulatory T cells were prepared by amplifying and/or inducing (according to the method shown in Example 7) such regulatory T cells using 4C8 mAb from CD4-positive T cells that had been separated by the method described in Example 2 from PBMC of the same donor. A group to which 1 × 10⁷ or 2 × 10⁷ PBMCs alone were administered, a group to which 1 × 10⁷ or 2 × 10⁷ regulatory T cells alone were administered, or a group to which a mixture of 1 × 10⁷ PBMCs and 1 × 10⁷ regulatory T cells (total 2 × 10⁷ cells) were intraperitoneally administered was provided. In addition to these groups, a group to which no cells had been transferred was provided. Furthermore, the transferred PBMCs were stained with anti-CD3 antibodies (Pharmingen) and then analysed using a FACSCalibur flow cytometer (Becton Dickinson). Thus, it was confirmed that the proportion of the CD3-positive T cells included in the PBMCs was 42%. 5 mice were used for each group. Observations of body weight and conditionwere carried out for 30 days after the transfer of the cells.

It has been previously confirmed that when human PBMCs are intraperitoneally administered to SCID mice to which TM-β1 antibodies have been administered, human T cells are activated to proliferate within the mouse bodies. The mice thus develop the symptoms of (hetero) graft versus host disease and then become weak and die. In this experiment, the mice (all the cases) of any group to which 1 × 10⁷ or 2 × 10⁷ PBMCs alone had been transferred died (Fig. 15) within approximately 2 weeks without recovering (Fig. 14) from transient body weight reduction due to irradiation with X-rays performed at the beginning of the experiment. On the other hand, regarding the mice to which 1 × 10⁷ or 2 × 10⁷ regulatory T cells alone had been transferred, no death cases were observed and all the mice (all the cases) survived (Fig. 15) until the completion of a 30-day observation period while showing body-weight change (Fig. 14) almost entirely similar to those in the group to which no cells had been administered. In the group to which the mixture of PBMCs and the regulatory T cells had been administered, 3 out of 5 mice (cases) died at a time somewhat later than those in the group to which PBMCs alone had been transferred. However, the remaining 2 out of 5 mice (cases) survived until the completion of the observation period and showed significantly prolonged survival time (based on a Logrank test) compared with all of the groups to which PBMCs alone had been administered (Fig. 15).

### Example 8-2: Suppression of lethality against mouse due to administration of human peripheral blood to SCID mouse by simultaneous administration of regulatory T cell and in vivo safety of regulatory T cell

To suppress the activity of NK cells so as to facilitate adhesion and integration of human cells in SCID mice (8-week-old, female, obtained from CLEA Japan, Inc.,), TM-β1 antibodies (20 µg/mouse, Pharmingen) that recognize IL-2β receptors were intraperitoneally administered on the day before the administration of the human cells. The mice were irradiated with a sublethal dose (2.5Gy) of X-rays on the day of administration of the human cells. The human cells were then administered. Leukapheresis sample from a healthy adult was subjected to specific gravity centrifugation using Lymphoprep (AXIS-SHIELD). Regulatory T cells were prepared by amplification and/or induction with Campath-1H (see Example 7-2) from the thus obtained mononuclear cells (PBMCs) or CD4-positive cells isolated by the method described in Example 2 from PBMCs of the same donor. A group to which 1.2 × 10⁷ PBMCs alone were administered, a group to which 1.2 × 10⁷ regulatory T cells alone were administered, a group to which the mixture of 1.2 × 10⁷ PBMCs and 1.2 × 10⁷ regulatory T cells (total 2.4 × 10⁷ cells) were intraperitoneally administered, and a group to which no cells were administered were provided. Except for the group to which regulatory T cells alone had been administered, the mice of each group were divided into mice of a group that were autopsied on day 11 after administration of the cells for observation of symptoms (including tissue findings) at the acute phase and mice of a group that were subjected to 21 days of observation concerning measurement of body weights and conditions. Five mice were used for each group. In addition, the mice (2 mice) of the group to which regulatory T cells alone had been administered were subjected only to autopsy on day 12 after administration of the cells because of the number of the obtained cells. Furthermore, it had been previously confirmed that the proportion of CD3-positive T cells contained in the administered PBMCs was 49.4% by staining of the T cells with anti-CD3 antibodies (Pharmingen) and then analysing using a FACSCalibur flow cytometer (Becton Dickinson).

We have previously confirmed that when human PBMCs are intraperitoneally administered to SCID mice to which TM-β1 antibodies have been administered, human T cells are activated to proliferate within the mouse bodies. The mice thus develop the symptoms of (hetero) graft versus host disease and then become weak and die. In this experiment, the mice (all the cases) of any group to which PBMCs alone had been administered died by day 21 (Fig. 17) without recovering (Fig. 16) from transient body weight reduction due to irradiation with X-rays at the time of the start of this experiment. In the case of the mice of the group to which the mixture of PBMCs and regulatory T cells had been administered, although total number of such human cells administered was simply twice the number of human cells administered in other cases, the group showed body weight fluctuations almost entirely similar to those in the group to which no cells had been administered (Fig. 16). All the mice (all the cases) of this group survived (Fig. 17). Furthermore, the mice to which regulatory T cells alone had been administered showed a recovery trend of body weight gain above that of the group to which no cells had been administered (Fig. 16). It was thus suggested that acute-phase invasion is merely developed against mice. Moreover, at the time of autopsy during the experimental period, infiltration with mononuclear cells, edema, dilatation of blood capillaries, and the like in the digestive tract were observed in the group to which PBMCs alone had been administered. However, no such changes were observed in the group to which the mixture of PBMCs and regulatory T cells had been administered and the group to which regulatory T cells alone had been administered (Fig. 18).

It was revealed by the above results that unlike PBMCs, regulatory T cells alone in this *in vivo* model system exert completely no pathogenicity against mice. It was also revealed that lethality against mice due to administration of PBMCs alone is significantly suppressed by simultaneous administration of regulatory T cells. These results further suggested that regulatory T cells induced by anti-CD52 antibodies suppress excessive *in vivo* activation of human T cells so that the administration thereof can be an effective therapy against various types of disease that develop accompanying abnormal activation of T cells.

All publications, patents, and patent applications cited herein are incorporated herein by reference in their entirety.

### Industrial Applicability

According to the present invention, it has been revealed that signals mediated by CD52 induce immunosuppression: specifically, differentiation induction and/or proliferation promotion for regulatory T cells and suppression of transendothelial cell migration. CD52 agonists, including antibodies against CD52, are useful as active ingredients of pharmaceutical compositions for immunosuppression, inducing differentiation and/or promoting proliferation of regulatory T cells, and suppressing transendothelial cell migration. Such compositions include pharmaceutical compositions for autoimmune disease and suppression of allergic disease, immune responses to transplantation, and the like. Furthermore, the present invention enables *ex vivo* preparation of regulatory T cells using the CD52 agonist and thus enables the utilization of such cells for treating and/or preventing autoimmune disease and symptoms and/or disease for which immunosuppression such as suppression of immune responses to transplantation is desired.

Through the use of regulatory T cells having antigen selectivity as shown by the present invention, suppression of a target antigen-specific immune reaction alone while avoiding side effects accompanying suppression of the entire immune system, which may take place by the use of conventional immunosuppressive agents can be expected.

## Claims

1. A pharmaceutical composition for immunosuppression, which contains as an active ingredient a CD52 agonist other than a 4C8 antibody.

2. A pharmaceutical composition for inducing differentiation and/or promoting proliferation of a regulatory T cell, which contains as an active ingredient a CD52 agonist other than a 4C8 antibody.

3. The pharmaceutical composition according to claim 1 or 2, wherein the regulatory T cell has antigen-selective suppressive activity.

4. The pharmaceutical composition according to any one of claims 1 to 3, which further contains a CD3 agonist.

5. The pharmaceutical composition according to claim 4, wherein the CD3 agonist is an anti-CD3 antibody or a fragment thereof.

6. The pharmaceutical composition according to claim 5, wherein the anti-CD3 antibody is a humanized antibody or a human antibody.

7. A pharmaceutical composition for suppressing transendothelial cell migration of an immunocyte, which contains as an active ingredient a CD52 agonist other than a 4C8 antibody.

8. The pharmaceutical composition according to any one of claims 1 to 7, wherein the CD52 agonist is an anti-CD52 antibody or a fragment thereof.

9. The pharmaceutical composition according to claim 8, wherein the anti-CD52 antibody is a humanized antibody or a human antibody.

10. The pharmaceutical composition according to claim 9, wherein the above humanized antibody is a rat humanized antibody Campath-1H.

11. The pharmaceutical composition according to any one of claims 1 to 10, which is used for preventing or treating autoimmune disease, allergic disease, or transplantation immune response.

12. A method for inducing differentiation and/or promoting proliferation of a regulatory T cell, which comprises causing a CD52 agonist other than a 4C8 antibody to act on CD52 that is expressed on the surface of an immunocyte;

13. The method for inducing differentiation and/or promoting proliferation of a regulatory T cell according to claim 12, wherein the regulatory T cell has antigen-selective suppressive activity.

14. The method according to claim 12, wherein the CD52 agonist is an anti-CD52 antibody or a fragment thereof.

15. The method according to claim 14, wherein the anti-CD52 antibody is a humanized antibody or a human antibody.

16. The method according to claim 15, wherein the above humanized antibody is the rat humanized antibody Campath-1H.

17. The method according to claim 12, which further comprises causing a CD3 agonist to act on CD3 that is expressed on the surface of the above immunocyte.

18. The method according to claim 17, wherein the CD3 agonist is an anti-CD3 antibody or a fragment thereof;

19. The method according to claim 18, wherein the anti-CD3 antibody is a humanized antibody or a human antibody.

20. The method according to any one of claims 12 to 19, wherein the above immunocyte is contained in peripheral blood, lymph node, or thymus.

21. The method according to claim 20, wherein the above immunocyte is a T cell.

22. The method according to claim 21, wherein the above immunocyte is a peripheral blood mononuclear cell.

23. The method according to any one of claims 12 to 22, wherein stimulation of an immunocyte with the CD3 agonist and the stimulation of an immunocyte with the CD52 agonist are carried out *ex vivo.*

24. The method according to any one of claims 12 to 22, wherein stimulation of an immunocyte with the CD3 agonist and stimulation of an immunocyte with the CD52 agonist are carried out *in vivo.*

25. A method for producing an anti-CD52 humanized antibody or human antibody to be used for a drug having an immunosuppressive effect, an effect of inducing differentiation and/or promoting proliferation of a regulatory T cell, and/or an effect of suppressing transendothelial cell migration of an immunocyte;

26. A method for screening using interaction with CD52 as an index for a drug having an immunosuppressive effect, an effect of inducing differentiation and/or promoting proliferation of a regulatory T cell, and/or an effect of suppressing transendothelial cell migration of an immunocyte.
